Europäisches Patentamt

European Patent Office

Office européen des brevets

(11) Publication number: **0 109 564**
**B1**

# (12) EUROPEAN PATENT SPECIFICATION

(45) Date of publication of patent specification: 27.04.88

(51) Int. Cl.⁴: **A 61 K 33/00,** A 61 K 33/24

(21) Application number: **83110490.6**

(22) Date of filing: **21.10.83**

(54) **The use of gallium salts for the manufacture of pharmaceutical compositions for the treatment of disorders of calcium homeostasis.**

(30) Priority: **22.10.82 US 436133**

(43) Date of publication of application:
**30.05.84 Bulletin 84/22**

(45) Publication of the grant of the patent:
**27.04.88 Bulletin 88/17**

(84) Designated Contracting States:
**DE FR GB IT**

(56) References cited:
**EP-A-0 059 148**

**CANCER CHEMOTHERAPY REPORTS, part 1, vol. 59, May/June 1975, R.H. ADAMSON: "Studies on the antitumor activity of gallium nitrate (NSC-15200) and other group IIIa metal salts", p. 599-610**

(73) Proprietor: **Sloan-Kettering Institute For Cancer Research**
**1275 York Avenue**
**New York New York 10021 (US)**

(72) Inventor: **Warrell, Raymond P.**
**300 East 54th Street**
**New York New York 10022 (US)**
Inventor: **Bockman, Richard**
**180 East 79th Street**
**New York New York 10021 (US)**

(74) Representative: **Patentanwälte Schulze Horn und Hoffmeister**
**Goldstrasse 36**
**D-4400 Münster (DE)**

Courier Press, Leamington Spa, England.

**Description**

This invention concerns the use of pharmaceutically acceptable gallium salts for the manufacture of pharmaceutical compositions or medicaments for preventing and treating disorders associated with excessive loss of calcium from bone.

Strict regulation of calcium ion concentration is necessary for normal cell functioning. In addition to maintaining cell membrane structure and excitability, calcium acts as a key second messenger to modulate critical biochemical pathways. Though intracellular levels of calcium are orders of magnitude less than extracellular levels, they reflect and depend on extracellular levels.

The calcium reservoir in the body is maintained in dynamic equilibrium between various tissues by a complex system of interrelated factors. Calcium is deposited in the bone but it can also be resorbed from bone and enter the mobile calcium pool in blood where it is carried to and from individual cells that require calcium. Ingested calcium replaces that lost by excretion, so that the amount of calcium in the pool normally remains constant. However, when one of the factors maintaining equilibrium fails, abnormalities in the concentration of calcium in specific tissues may result, Hypercalcemia is one such abnormal state.

Hypercalcemia raises intracellular levels of calcium which results in profound disruption of physiological processes within all cells. In the earliest phases, few signs or symptoms are present. With rising serum calcium levels (11 to 13 mg%) polyuria and nocturia associated with nausea, anorexia, fatigue, constipation, and mental dullness may develop. With higher serum calcium levels (>13 mg%), decreased mentation progresses to obtundation and ileus develops. Furthermore, complete cardiovascular collapse can result from several factors: dehydration caused by water loss associated with calciuresis, the renal tubular defect in conserving water, chronic emesis, and decreased fluid intake. In rare instances, the positive inotropic effect of calcium on the cardiac muscle has even been reported to trigger serious arrhythmias.

Diseases associated with hypercalcemia include a wide variety of human cancers as well as primary hyperparathyroidism. Both of the latter diseases are extremely prevalent and thus provide the urgency to develop an effective form of therapy.

Cancer-associated hypercalcemia is being recognized as one of the most commonly occurring paraneoplastic syndromes. This morbidity of cancer may arise suddenly and can progress rapidly, resulting in a life-threatening metabolic disturbance requiring prompt and effective treatment.

Unfortunately, most therapies in current use are non-specific and are not directed at the underlying pathophysiological processes that cause increased bone resorption and calcium release.

Among the recommended treatment choices in hypercalcemia are calciuretic diuretics such as ethacrynic acid or furosemide (Lasix) glucocorticoid steroids or, in the case of patients with predictable and progressive hypercalcemia IV infusion of mithramycin (Bockman, R., Your Patients and Cancer, March, 1—5 (1981)).

Accordingly, a search has been undertaken to discover agents which inhibit calcium resorption from bone. Accordingly, it is the principal object of the present invention to provide pharmaceutical compositions for regulating resorption of calcium from bone, especially to provide a medicament for treating disorders associated with excessive loss of calcium from bone.

It is another object of the present invention to provide a medicament to prevent and to treat hypercalcemia associated with cancer or hyperparathyroidism, the two most common causes, and further to prevent osteopenia due to calcium resorption from bone.

It has been unexpectedly observed that gallium salts are effective regulators of calcium resorption from bone and are effective in the treatment of hypercalcemia associated with cancers. The invention refers to the use of pharmaceutically acceptable gallium salts for the manufacture of pharmaceutical compositions or medicaments for preventing or treating disorders associated with excessive loss of calcium from bone.

A further effect of the present invention is to provide an in-vitro-method of inhibiting the release of calcium from cultured bone cells by contacting said culture cells with a pharmaceutically acceptable form of gallium salts.

In accordance with the present invention gallium salts are administered in non-nephrotoxic amounts to inhibit resorption of calcium from bone in patients with hypercalcemia, bone fragility or other disorders associated with abnormally increased calcium resorption.

Gallium is a metal belonging to the group IIIa elements of the periodic table. In 1950, gallium-72 was found to accumulate in neoplastic tissues (Dudley, H. C. *et al.*, Radiology, *50*, 571 (1950), and shortly thereafter, the therapeutic use of radioactive gallium was first reported by King *et al.*, for the treatment of patients with malignant bone tumors (King, E. R., *et al.*, Arch Int. Med., *90*, 785 (1952)). In 1969, Edwards and Hays observed that carrier-free gallium-67 localized at sites of tumors in cancer patients (Edwards, C. L., *et al.*, J. Nuc. Med., *10*, 103 (1969)). Since then, gallium-67 citrate has been widely used as an oncophilic agent for the diagnostic evaluation of patients with a variety of neoplastic diseases, particularly malignant lymphoma (McCaffrey, J. A. *et al.*, Am. J. Med., *60*, 523 (1976); Hoffer, P., J. Nuc. Med. *21*, 394 (1980)).

Further, studies on the antitumor activities of gallium nitrate (Adamson, R. H., *et al.*, Cancer Chemotherapy Reports, Part 1, Vol. 59 (1975), pp. 599—609) with rodents demonstrated, that gallium nitrate was active against said tumors. Also, the use of gallium chloride has been described as a medicament for treating maligneous tumors (EP—A—59 148).

While conducting clinical trials with gallium nitrate as an anti-cancer agent, it has been observed by the applicant that blood calcium levels frequently dropped to subnormal levels. Previously it had been reported that gallium nitrate caused excess urinary excretion of calcium (hypercalciuria), when administered with mannitol (Krakoff, *et al.,* Cancer, *44*, 1722 (1979)).

However, our later carefully controlled metabolic studies showed that this observation was an artifact probably caused by the diuretic effect of mannitol. In our metabolic studies, the net amount of calcium was calculated from measurements of intake and excretion. This monitoring of calcium showed that gallium nitrate did not cause any change in calcium excretion even though it reduced blood levels of calcium. This suggests that gallium ion is exerting its hypocalcemic effect by inhibiting an important factor which maintains the normal dynamic equilibrium of the calcium pool *i.e.,* bone resorption. That the equilibrium involved that of bone resorption was confirmed by later experiments with bone cell cultures wherein it was shown that loss of calcium from bone tissue of fetal rats was inhibited by gallium nitrate even in the presence of a potent calcium-releasing agent, OAF.

Gallium salts which may be employed are those which are physiologically acceptable including nitrate, citrate, halide, preferably chloride, carbonate, acetate, tartrate, oxalate, oxide or hydrated gallium oxide. It is to be understood that the active therapeutic agent is the gallium ion and, that therefore the choice of anion may be determined by such factors as commercial accessibility, solubility characteristics, and mode of administration.

The gallium salt may be administered orally or intravenously. In the preferred embodiment of the present invention, gallium nitrate is administered by continuous infusion.

The hypocalcemic effect of gallium salts is strictly schedule related. Previous clinical trials of gallium nitrate demonstrated that bolus administration produced dose limiting nephrotoxicity. In an effort to increase the therapeutic index of this compound and to establish a satisfactory out-patient schedule which avoided the use of parenteral hydration, the therapeutic and toxic effects of gallium nitrate administered as a continuous infusion were evaluated. It was found that gallium nitrate administered as a continuous infusion for seven days at 200 to 400 mg/m$^2$/day is well tolerated and is an effective treatment for patients with hypercalcemia. Out-patient administration using portable infusion pumps is safe and practical. It is expected that oral administration of gallium salts will also be effective especially when used in conjunction with time release coatings which allow a controlled supply of gallium to the bloodstream.


Metabolic Studies on Patients  Receiving Gallium Salts

Studies of calcium homeostasis require an assessment of multiple factors including dietary intake, excretion of calcium into urine and stool, and analysis of calcium mobilization from body stores (chief bone). A series of patients who participated in a careful study of the effects of gallium nitrate upon calcium metabolism were thus assessed. Patients were hospitalized and received a diet of defined calcium, sodium, and fluid intake. Measurements of calcium excretion into urine and stool (along with multiple other laboratory tests) were made daily during a 6-day baseline period and during a subsequent treatment period using gallium nitrate. The drug was administered as a continuous infusion at a dose of 300 mg/m$^2$/day for durations ranging from 4—7 days. Despite the finding that the total serum calcium concentration was reduced by the gallium infusion, we found no significant increase in calcium excretion during the infusion relative to the baseline observation period. These data suggested that gallium nitrate might exert its hypocalcemic effect by directly affecting calcium resorption from bone.


In Vitro Effects of Gallium Nitrate Upon Bone Resorption

Using an *in vitro* assay of fetal rat bones (Bockman, R. S and Repo, M.A., J. Exp. Med. *154* 529 (1981)), the effects of gallium nitrate upon calcium release from bone were measured after exposure to a tumor-derived factor (osteoclast activating factor, OAF). Simultaneous addition of gallium and OAF did *not* reduce calcium release relative to the use of OAF alone. However, pre-incubation of bones with gallium for 48 hours preceding the addition of OAF caused a marked reduction in calcium release. Moreover, at the doses of gallium nitrate employed in these tests (1—10 µg/ml), no toxic effect on bone metabolism was observed. These data suggested a unique mechanism of gallium-nitrate induced hypocalcemia, namely a direct inhibition of calcium resorption from bone which was independent of any cytotoxic effect. This mechanism also indicates that gallium might prove useful for the treatment of diseases associated with increased losses of calcium from bone.


Treatment of Cancer-Related Hypercalcemia With Gallium Nitrate Infusions

Gallium nitrate was used for the treatment of seven patients with cancer-related hypercalcemia. The daily dose of drug was 200 mg/m$^2$ administered as a continuous infusion for durations ranging from 5—7 days. The diagnoses of this patient population and the change in total serum calcium concentration in response to this therapy are presented in Table 1. Note that *all* patients responded to this treatment by a reduction in serum calcium concentration to normal (and frequently sub-normal) values.

With the single exception of Patient 5 (Table 1), the hypocalcemic effect was not associated with any anti-cancer effect. Patient 5 demonstrated a transient decrease in the size of a lymphomatous mass

3

followed shortly thereafter by progression of her disease and death. The subsequent increase of her disease was not accompanied by an increase in serum calcium which indicates a persistent control of the metabolic problem by the previous treatment with gallium nitrate. All other patients who received the drug manifested progressive cancer despite control of the hypercalcemia. This finding indicates that the hypocalcemic effects is not produced by a direct cytotoxic effect of gallium nitrate upon tumor cells.

TABLE 1
Response of patients with cancer-related hypercalcemia
to continuous infusion of gallium nitrate

| Patient | Cancer Diagnosis | Total Serum Calcium[1] | | Daily Dose of Gallium Nitrate |
| | | Pre-Treatment | Post-Treatment | |
| --- | --- | --- | --- | --- |
| | | | | $(mg/m^2) \times$ days |
| 1 | Breast | 13.6 | 8.9 | $200 \times 5$ |
| 2 | Lymphoma | 14.0 | 6.7 | $200 \times 6$ |
| 3 | Head & Neck | 12.3 | 8.5 | $200 \times 5$ |
| 4 | Breast | 14.4 | 7.7 | $200 \times 6$ |
| 5 | Lumphoma | 15.6 | 8.6 | $200 \times 7$ |
| 6 | Lung | 12.5 | 7.0 | $200 \times 5$ |
| 7 | Lung | 12.7 | 9.4 | $200 \times 5$ |

[1] Serum concentration expressed in mg/dl (formal range, 9.0—10.8 mg/dl).

Recent studies (Warrell R. P., *et al.,* Cancer 51:1982—87, 1983) have strongly suggested that administration of gallium nitrate by continuous intravenous infusion produces a substantial increase in the drug's therapeutic index as a cytotoxic agent, relative to administration as a bolus injection (i.e. more effective, less toxic). The infusion method avoids large, peak plasma drug concentrations and it achieves prolonged exposure to low, constant plasma drug levels. An oral method of administration would provide not only a convenient route of drug delivery but it would also allow one to achieve constant, low-level plasma drug concentrations and avoid large peak levels. Prolonged exposure (greater than 48 hours) to gallium nitrate concentrations which ranged from 1—10 µg/ml is necessary to inhibit stimulated bone resorption in vitro. As previous pharmacokinetic studies (Kelsen, D. P. *et al.,* Cancer 46:2009—13, 1980) have shown, gallium nitrate administration by continuous infusion at a daily dose of 200 mg/m²/day achieves a steady state plasma gallium concentration which ranges from 0.9—2.0 µg/ml. This dose is extremely effective in controlling cancer related hypercalcemia (see Table 1 of this application). Therefore, an orally administered gallium salt which achieves a plasma gallium concentration of approximately 0.9—2.0 µg/ml should also be effective in controlling cancer related hypercalcemia as well as other disorders associated with accelerated loss of calcium from bone.

In order to evaluate oral absorption of gallium salts, a concentrated solution of gallium nitrate was administered by oral gavage to a dog. The total oral dose was 1.200 mg. The next 24 hour urine specimen was then collected from the dog and assayed for gallium concentrations by atomic absorption spectrophotometry of the urine as well as of sequentially drawn plasma samples.

The 24 hour urine volume was 183 ml, and it contained a total amount of 1.13 mg of gallium. The blood levels achieved at various times after drug administration are noted in Table 2. Importantly, the animal who received this oral dose of gallium nitrate experienced no toxic reactions whatsoever.

TABLE 2
Plasma gallium concentrations
achieved in a dog
after administration of
1.200 mg gallium nitrate
by oral gavage

| time | gallium concentration (µg/ml) |
|---|---|
| 0 | 0 |
| 15 min | 0.5 |
| 32 min | 1.25 |
| 60 min | 1.37 |
| 120 min | 2.75 |
| 240 min | 2.0 |
| 24 h | 0.75 |

Approximately two-thirds of an intravenously injected dose of gallium nitrate is excreted into the urine within the first 24 hours (Kelson, D. P., et al., Cancer 46:2009—13, 1980). Based on the reasonable assumption that excretion of gallium is similar in dogs and humans, one can conclude from the urinary data that approximately 1—2 percent of an orally administered dose of gallium nitrate is absorbed and subsequently excreted into urine. Moreover, oral administration of a 1200 mg dose of gallium nitrate achieved a sustained plasma gallium concentration at a level known to be effective in inhibiting bone resorption in vitro and in controlling cancer-related hypercalcemia in vivo. Administration of daily or twice daily oral doses would reasonably be expected to maintain a constant, low level plasma gallium concentration. If a 1200 mg dose of gallium nitrate achieves a pharmacologically active concentration of gallium in the plasma of a 10 kg dog, it is estimated that 0.5—20 g of gallium nitrate administered orally to a 70 kg human would achieve similarly therapeutic concentrations of gallium in plasma. Depending upon differences in possible pharmacodynamic behavior between species. An oral dose in humans of this magnitude administered 1—4 times/day is anticipated.

**Claims**

1. Use of pharmaceutically acceptable gallium salts for the manufacture of pharmaceutical compositions or medicaments for preventing or treating disorders associated with excessive loss of calcium from bone.

2. Use according to claim 1, characterized in that said compositions of medicaments comprise a gallium salt from the group of gallium nitrate, gallium citrate, gallium halide, preferably chloride, gallium carbonate, gallium acetate, gallium tartrate, gallium oxalate, gallium oxide and hydrated gallium oxide.

3. Use according to claim 1, characterized in that said disorder is hypercalcemia.

4. Use according to claim 1, characterized in that said disorder is osteopenia.

5. Use according to claim 1, characterized in that said disorder is bone fragility.

6. Use according to claim 1 or 2, characterized in that said compositions are in a form suitable for continuous intraveous infusion.

7. Use according to claim 1 or 2, characterized in that said compositions are in a form suitable for oral administration.

8. In-vitro-method of inhibiting the release of calcium from cultured bone cells by contacting said culture cells with a pharmaceutically acceptable form of gallium salts.

**Patentansprüche**

1. Verwendung von pharmazeutisch anwendbaren Gallium-Salzen zur Herstellung von pharmazeutischen Zusamensetzungen oder Arzneimitteln für die Verhinderung oder Behandlung von Disfunktionen, die mit einem übermäßigen Verlust von Kalzium aus Knochen verbunden sind.

2. Verwendung entsprechend Anspruch 1, dadurch gekennzeichnet, daß die genannten Zusammensetzungen oder Arzneimittel ein Gallium-Salz aus der Gruppe:

Gallium-Nitrat, Gallium-Citrat, Gallium-Halogenid, vorzugsweise Chlorid, Gallium-Karbonat, Gallium-Acetat, Gallium-Tartrat, Gallium-Oxalat, Gallium-Oxid und hydriertes Gallium-Oxid, enthalten.

3. Verwendung entsprechend Anspruch 1, dadurch gekennzeichnet, daß die genannte Disfunktion Hyperkalzämie ist.

4. Verwendung entsprechend Anspruch 1, dadurch gekennzeichnet, daß die genannte Disfunktion Osteopenie ist.

5. Verwendung entsprechend Anspruch 1, dadurch gekennzeichnet, daß die genannte Disfunktion Knochenfragilität ist.

6. Verwendung entsprechend Anspruch 1 oder 2, dadurch gekennzeichnet, daß die genannten Zusammensetzungen eine für eine kontinuierliche, intravenöse Infusion geeignete Form haben.

7. Verwendung entsprechend Anspruch 1 oder 2, dadurch gekennzeichnet, daß die genannten Zusammensetzungen eine für die orale Darreichung geeignete Form haben.

8. In-vitro-Methode einer Hemmung der Freisetzung von Kalzium aus kultivierten Knochenzellen durch Kontakt der genannten Kulturzellen mit einer pharmazeutisch akzeptablen Form von Gallium-Salzen.

**Revendications**

1. Utilisation de sels de gallium pharmaceutiquement acceptables pour la fabrication de compositions pharmaceutiques ou de médicaments pour éviter ou traiter les troubles associés à une perte excessive de calcium de l'os.

2. Utilisation suivant la revendication 1, caractérisée en ce que lesdites compositions ou lesdits médicaments comprennent un sel de gallium choisi parmi nitrate de gallium, citrate de gallium, halogénure de gallium, de préférence chlorure, carbonate de gallium, acétate de gallium, tartrate de gallium, oxalate de gallium, oxyde de gallium et oxyde hydraté de gallium.

3. Utilisation suivant la revendication 1, caractérisée en ce que ledit trouble est l'hypercalcémie.

4. Utilisation suivant la revendication 1, caractérisée en ce que ledit trouble est l'ostéopénie.

5. Utilisation suivant la revendication 1, caractérisée en ce que ledit trouble est la fragilité des os.

6. Utilisation suivant la revendication 1 ou 2, caractérisée en ce que lesdites compositions sont sous une forme appropriée pour une perfusion intraveineuse continue.

7. Utilisation suivant la revendication 1 ou 2, caractérisée en ce que lesdites compositions sont soue une forme appropriée pour une administration orale.

8. Méthode in vitro d'inhibition de la libération du calcium de cellules d'os en culture, par mise en contact desdites cellules de culture avec une forme pharmaceutiquement acceptable de sels de gallium.